Europäisches Patentamt

European Patent Office

Office européen des brevets

(19)

(11) Publication number: 0 146 992

A1

(12) **EUROPEAN PATENT APPLICATION**

(21) Application number: 84201846.7

(22) Date of filing: 12.12.84

(51) Int. Cl.⁴: **A 61 B 6/00**
**G 21 K 1/10**

(30) Priority: 22.12.83 NL 8304398

(43) Date of publication of application:
03.07.85 Bulletin 85/27

(84) Designated Contracting States:
DE FR GB IT NL

(71) Applicant: N.V. Philips' Gloeilampenfabrieken
Groenewoudseweg 1
NL-5621 BA Eindhoven(NL)

(72) Inventor: Janssen, Jozef theodorus Antonius
c/o INT. OCTROOIBUREAU B.V. Prof. Holstlaan 6
NL-5656 AA Eindhoven(NL)

(72) Inventor: Knibbe, Gerard
c/o INT. OCTROOIBUREAU B.V. Prof. Holstlaan 6
NL-5656 AA Eindhoven(NL)

(74) Representative: Scheele, Edial François et al,
INTERNATIONAAL OCTROOIBUREAU B.V. Prof.
Holstlaan 6
NL-5656 AA Eindhoven(NL)

(54) X-ray examination apparatus having a selective filter.

(57) An X-ray examination apparatus for examination of composite objects, such as, for example, peripheral examination of patients, comprises, in order to avoid over-radiation of the detection system by radiation passing along the object, a filter that can be adapted to the interstices.

Such a filter consists, for example, of a body of radiation-absorbing material having in two directions a varying radiation absorption, for example in the form of a double wedge.

FIG.2

X-ray examination apparatus having a selective filter.

The invention relates to an X-ray examination apparatus for peripheral examination comprising an X-ray source, an object table and an X-ray detection system.

In such apparatus, during the image formation a disturbing effect may be exerted by radiation which passes without hindrance along an object or _via_ interstices in a composite object such as, for example, both legs of a patient. Such a radiation disturbs an image to be formed by local strong overexposure, which impedes the optimization of the detection system and the use of an automatic exposure control during the image formation.

The invention has for its object to obviate these disadvantages and according to the invention an X-ray examination apparatus of the kind mentioned in the opening paragraph is for this purpose characterized in that, in order to avoid overradiation of the detection system by radiation passing through the object space in substantially unattennated form, there is arranged a radiation filter having a variable radiation absorption in two orthogonal directions.

Due to the fact that in an X-ray examination apparatus, for example for angiographic examination of the legs of a patient, by means of a filter of the kind described, the space between the legs can be locally screened from over-radiation, a substantially better image formation is possible. Such an improvement can also be obtained by a filter adapted to an outer boundary of an object.

In a preferred embodiment, the filter is transversely displaceable in the radiation beam for adaption to the form of the space to be screened in the image-forming device.

The fact that the filter as such is also imaged does not lead to a great disadvantage because at the area of the filter no relevant information for the examination, for example angiographic examination of the legs of a patient, is present as yet. Such a possible disadvantage is completely avoided with the use in an X-ray examination apparatus equipped with a device for, for example, digital image subtraction. In fact, in this case the the image of the filter just disappears from the ultimage image due to the subtraction.

In X-ray examination apparatus according to the invention, the use of automatic exposure control is possible with a high accuracy due to the absence of the direct radiation, as a result of which the image formation can be improved and the radiation dose can be reduced, respectively.

A filter for an X-ray examination apparatus according to the invention consists, for example, of a body of X-ray radiation absorbing material, such as, for example, aluminium, which extends in two orthogonal planes in the form of a wedge. A stronger variation of the absorption can be realized in that a part of the wedge of a material having a comparatively low atomic number is replaced by a wedge of a material having a higher atomic number, preferably so that the radiation absorption for the two materials differs by about a factor two or more. Especially when a filter is formed, for example, as a mould, the variation of the radiation absorption in each of the two directions can be adapted by the shape and by materials to be used to object types to be measured.

A few preferred embodiments according to the invention will now be described more fully with reference to the drawing. In the drawing:

Figure 1 shows an X-ray examination apparatus according to the invention, and

Figure 2 shows an embodiment of a radiation filter thereof in plan view a, in cross-section of view b and in long itudinal sectional view c.

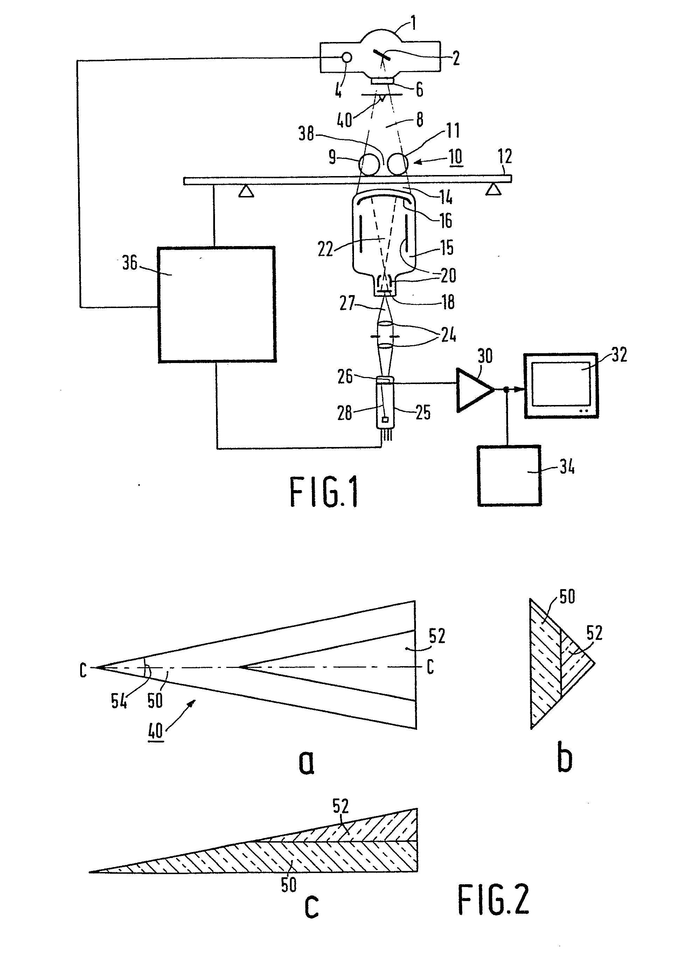

An X-ray examination apparatus of the kind shown in Figure 1 comprises an X-ray tube 1 having an anode 2, a cathode 4 and an exit window 6 for producing an X-ray beam 8, by which in this case a composite object 10, for example consisting of two adjacent legs 9 and 11 lying on an object table 12, can be exposed. On a side of the object table remote from X-ray source there is situated in the X-ray beam 14 now carrying image information an X-ray image intensifier tube 15 having an input screen 16, an output screen 18 and an electron-optical system 20 for imaging an electron beam 22 released by the X-ray beam 14 from a photocathode of the input screen on the output screen.

The output screen 18 is optically coupled by a fibre-optical system or as in this case, by a lens system 24 with an input screen 26 of a television camera tube 25. In the television camera tube, an image projected by a light beam 27 onto the input screen 26 thereof is scanned by means of an electron beam 28 and is converted into a video signal. The whole detection system with the X-ray image intensifier tube and the television camera tube may be replaced by a film camera or a film cassette. The images are then recorded, for example, via an X-ray intensifier screen directly on a film. The X-ray tube with the detection system 29 constitutes together with the object carrier 12 parts of, for example, the X-ray examination apparatus described in GB 2098440, in which the X-ray source 1 with the detection system 29 can be displaced in the manner described therein with respect to the object carrier. The video signal is supplied in this case via a preamplifier 30 to a monitor 32 and t a possibly present device 34 for digital image substraction. For mutual control and, where required, for synchronization of the television camera tube, the X-ray tube and the position of the object table and hence of the object, a central control device 36 is provided.

In order to reduce radiation, which would reach the detector without hindrance via an opening 38 between the two parts 9 and 11 of the composite object 10, a filter 40 is arranged in the radiation path. The filter 40 is placed, for example, against a collimator of the exit window not shown. For adaptation to the geometry of the opening 38, the filter is preferably arranged so as to be displaceable, in this case in a direction at right angles to the plane of the drawing, with respect to the exit window, so transversely to the main ray of the X-ray beam.

A filter of the kind shown in Figure 2 in two sectional views b and c and in plan view a consists of a flattened first wedge 50, for example of aluminium, on which is disposed a second wedge 52 of, for example, stainless steel or another material mainly of one or several elements having a comparatively high atomic number. A wedge thus composed has, for example, a length of 400 mm, a width of 50 mm and a height of about 25 mm. the second filter occupying, for example, 10 mm of this height. Both in the longitudinal direction and in the direction of width, a varying radiation absorption is thus obtained having in the direction of width a maximum at the centre of the filter. By variations in the ratio between width and height, the apical angle 54, the overall length the dimensions of the second filter with respect to the first filter, the materials to be used and the like, the filter can be adapted to various types of composite objects. In the arrangement shown in Figure 1, in which the sub-objects 9 and 11 represent the legs of the patient, the filter is mounted with the longitudinal axis at right angles to the plane of the drawing and is arranged there so as to be adjustable also in this direction for adaptation to the interstice 38.

With possible small adaptations, the filter may be used, for example also in the form of a set of filters composed of a double wedge form of one or several materials, in all the X-ray examination apparatus, by which at least also composite objects are examined. An example of an X-ray examination apparatus in which the filter can be used successfully is described in the co-pending Patent Application ... (PHN 10.882) (in the name of the Applicant).

1.          An X-ray examination apparatus comprising an X-ray source, an object table and an X-ray detection system, characterized in that, in order to avoid over-radiation of the detection system by radiation which passes through an object space in substantially imattennated form, there is provided a radiation filter having a variable radiation absorption in two orthogonal directions.

2.          An X-ray examination apparatus as claimed in Claim 1, characterized in that the filter has the form of a wedge in two orthogonal planes.

3.          An X-ray examination apparatus as claimed in Claim 2, characterized in that the filter extends in one plane as a single wedge and in one plane at right angles thereto as a double wedge.

4.          An X-ray examination apparatus as claimed in any one of the preceding Claims, characterized in that there is arranged centrally on a wedge form a second wedge form of radiation-absorbing material having a higher specific radiation absorption as compared with the material of the first wedge.

5.          An X-ray examination apparatus as claimed in Claim 4, characterized in that a first wedge consists mainly of an element having a comparatively low atomic number and the second wedge consists mainly of an element having a comparatively high atomic number.

6.          An X-ray examination apparatus as claimed in any one of the preceding Claims, characterized in that the radiation filter comprises at least two materials having a mutual difference in the absorption coefficient of about a factor two for the radiation to be used.

7.          An X-ray examination apparatus as claimed in any one of the preceding Claims, characterized in that the filter is arranged so as to be displaceable transversely to the direction of propagation of an X-ray beam to be emitted by the X-ray source with respect to the X-ray source.

8.          An X-ray examination apparatus as claimed in any one of the preceding Claims, characterized in that an automatic exposure control is included therein.

9.          An X-ray examination apparatus as claimed in any one of the preceding Claims, characterized in that a device for digital image subtraction is included therein.

10.          A wedge-shaped filter intended to be used in an X-ray examination apparatus as claimed in any one of the preceding Claims.

0146992

FIG.1

a

b

c

FIG.2

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | FR-A-2 485 790 (COMP. GEN. DE RADIOLOGIE) <br> * Page 2, lines 10-21; page 3, lines 18-32; page 4, line 18 - page 5, line 1; figures 1-3 * | 1,7,10 | A 61 B 6/00 <br> G 21 K 1/10 |
| | --- | | |
| A | GB-A-2 098 425 (BOARD OF TRUSTEES OF LELAND STANFORD JR. UNIVERSITY) <br> * Abstract; page 1, line 117 - page 2, line 13; page 2, lines 49-78; figures 1,2 * | 1,5,9, 10 | |
| | --- | | |
| A | CH-A- 254 461 (H. IMFELD) <br><br> * Page 2, lines 27-46, 60-69; figures 1-8 * | 1,3,7, 10 | |
| | --- | | TECHNICAL FIELDS SEARCHED (Int. Cl.4) |
| A | CH-A- 243 731 (H. IMFELD) <br><br> * Page 2, lines 7-51; figures 1-6 * | 1,3,7, 10 | A 61 B <br> G 21 K |
| | --- | | |
| A | DE-A-3 017 745 (VARIAN ASSOCIATES, INC.) <br> * Page 5, lines 13-22; page 7, line 20 - page 8, line 2; figures 3,4 * | 1,5,10 | |
| | ---     -/- | | |

The present search report has been drawn up for all claims

| Place of search | Date of completion of the search | Examiner |
|---|---|---|
| THE HAGUE | 27-03-1985 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | DE-A-1 930 283 (SIEMENS AG) * Page 1, lines 17-23; page 2, lines 19-27; page 3, lines 24-27; figure 1 * | 1,8,10 | |
| A | US-A-4 181 858 (J.F. MOORE) * Abstract; column 4, lines 24-39; column 6, lines 9-63; column 7, lines 42-56; figures 1a-6 * | 1,7,10 | |

TECHNICAL FIELDS SEARCHED (Int. Cl.4)

The present search report has been drawn up for all claims

| Place of search | Date of completion of the search | Examiner |
|---|---|---|
| THE HAGUE | 27-03-1985 | RIEB K.D. |